# FASCICULE DE BREVET EUROPEEN

(11) **EP 1 497 351 B1**
(45) Date de publication et mention de la délivrance du brevet: **08.10.2008**
(21) Numéro de dépôt: 03727617.7
(22) Date de dépôt: 27.03.2003
(51) Int. Cl.: C08G 18/79, C08G 18/02, C07D 251/34

(54) **COMPOSITION POLYISOCYANATE DE FAIBLE VISCOSITE POSSEDANT UNE FONCTIONNALITE ELEVEE ET PROCEDE DE PREPARTION**
NIEDRIGE VISKOSITÄT UND HOHE FUNKTIONALITÄT AUFWEISENDE POLYISOCYANATZUSAMMENSETZUNG UND VERFAHREN ZUR HERSTELLUNG
LOW-VISCOSITY POLYISOCYANATE COMPOSITION HAVING HIGH FUNCTIONALITY AND METHOD FOR PREPARING SAME

(30) Priorité: 27.03.2002 FR 0203877
(43) Date de publication de la demande: 19.01.2005
(73) Titulaire: RHODIA CHIMIE, 92510 Boulogne-Billancourt (FR)
(72) Inventeur: BERNARD, Jean-Marie, F-69440 MORNANT (FR)
(74) Mandataire: Colombet, Alain André
(86) Numéro de dépôt international: PCT/FR2003/000976
(87) Numéro de publication internationale: WO 2003/080697

(56) Documents cités:
- EP-A- 0 478 990
- EP-A- 0 645 411
- WO-A-99/07765
- DE-A- 19 603 245

## Description

L'invention concerne une composition polyisocyanate éventuellement masquée de relativement faible viscosité possédant une fonctionnalité moyenne élevée d'au moins 3 ou plus. L'invention concerne également un procédé de préparation d'une composition polylsocyanate de faible viscosité possédant une fonctionnalité moyenne élevée en groupes isocyanates.

Des compositions polyisocyanates masquées ou non possédant une fonctionnalité moyenne supérieure à 2 sont généralement obtenues par cyclotrimérisation catalytique de diisocyanates et contiennent des composés comprenant des groupes isocyanurate.

De telles compositions et procédés pour leur préparation sont décrits dans, par exemple, US 4,324,879 et US 4,412,073. Bien que ces compositions possèdent des propriétés remarquables, elles ont cependant une viscosité élevée qui nécessite leur dilution par des solvants organiques.

Une des solutions préconisées à cet effet est de stopper la réaction de cyclotrimérisation à un très faible degré de trimérisation pour augmenter la quantité de polyisocyanates monoisocyanurates et réduire la quantité de polyisocyanates isocyanurates possédant plus d'un cycle isocyanurate. Ainsi, le brevet US 4,801,663 ou encore la demande de brevet WO-A-99/07765 décrit un procédé de cyclotrimérisation d'1,6-hexaméthylène dilsocyanate (HDI) dans lequel la trimérisation est arrêtée à un faible taux de transformation.

L'inconvénient d'un tel procédé est cependant une diminution importante du rendement global de la réaction qui nécessite d'éliminer du produit réactionnel final une quantité importante de monomères n'ayant pas réagi, ce qui a pour effet d'augmenter considérablement le coût du procédé.
La demande de brevet EP-A-0 645 411 divulgue un agent de réticulation de type polyisocyanate de faible viscosité, comprenant des motifs urétidine-diones et des motifs isocyanusates, obtenus par modification de groupements urethane de monomères diisocyanate avec un diol.

Une autre solution proposée (cf. US 5,750,629) consiste à soumettre les monomères isocyanates à une réaction de cyclodimérisation avant, après ou pendant l'étape de cyclotrimérisation pour aboutir à une composition polyisocyanate possédant des composés isocyanates à groupes isocyanurates et des composés isocyanates à groupes urétidinediones (1,3-diazétidine-2,4-diones), notamment des composés mono-urétidinediones.

L'inconvénient de ce procédé est toutefois une diminution de la fonctionnalité moyenne en groupes NCO du produit réactionnel en raison d'une proportion plus ou moins élevée de composés urétidinediones.

Une autre solution préconisée dans US 4,810,820 consiste à ajouter au mélange réactionnel un alcool avant, après ou pendant la réaction de trimérisation pour obtenir un mélange polyisocyanate comportant des groupes isocyanurates et des groupes allophanates. Toutefois, comme précédemment, l'inconvénient de cette méthode réside en une diminution de la fonctionnalité de la composition polyisocyanate finale, notamment en raison de la présence d'allophanates « vrais », composés constitués de deux chaînes monomères d'isocyanates et d'une fonction allophanates, dont la fonctionnalité est de 2 dans le cas de diisocyanates, ou de polyallophanates.

Ainsi, un but de la présente invention est de fournir une composition polyisocyanate, éventuellement masquée, de viscosité réduite possédant une fonctionnalité élevée d'au moins 3, de préférence supérieure à 3, dans le cas où elle est obtenue par polycondensation de diisocyanates et une fonctionnalité plus élevée encore dans le cas de triisocyanates.

Un autre but de la présente invention est de fournir une composition polyisocyanate, éventuellement masquée, de viscosité réduite, possédant une teneur relativement faible en polyisocyanates mono-urétidinedione, et dont le taux de transformation des monomères de départ est supérieur à 35%.

Un autre but encore de la présente invention est de fournir une composition polyisocyanate éventuellement masquée de viscosité réduite possédant une teneur réduite en polyisocyanates comprenant des groupes isocyanurates, notamment de composés mono-isocyanurates, celle-ci étant avantageusement non supérieure à 45 %, de préférence non supérieure à 40 % en poids, par rapport au poids des isocyanates de la composition polyisocyanate.

Ces buts sont atteints grâce à la présente invention qui a pour objet une composition polyisocyanate ayant une fonctionnalité moyenne élevée, obtenue par polycondensation de monomères diisocyanates ou triisocyanates, comportant :
(a) de 0,5 à 30 % en masse par rapport à la masse totale des composants a), b) et c) de composés porteurs d'une seule fonction urétidinedione ayant une masse moléculaire au plus égale à deux fois la masse moléculaire moyenne des monomères isocyanates ayant la masse moléculaire la plus élevée ;
(b) de 0,5 à 45 % en masse par rapport à la masse totale des composants a), b) et c) de composés porteurs d'une seule fonction isocyanurate et de masse moléculaire au plus égale à trois fois la masse moléculaire moyenne desdits monomères isocyanates ayant la masse moléculaire la plus élevée ; le rapport molaire de (a)/(b) étant compris entre 0,02 et 2, avantageusement entre 0,2 et 1,8, de préférence inférieur ou égal à 1,6,
(c) au moins 40 % en masse par rapport à la masse totale des composants a), b) et c) d'un mélange de composés polyisocyanates présentant une masse moléculaire au moins égale à trois fois la masse moléculaire moyenne des monomères isocyanates ayant la masse moléculaire la plus petite et porteurs d'au moins deux fonctions isocyanates, et
   ledit mélange comportant :
   (i) des composés porteurs d'au moins deux fonctions isocyanurates, à l'exclusion de ceux qui comportent des fonctions urétidinediones,
   (ii) des composés porteurs d'au moins deux fonctions urétidinediones, à l'exclusion de ceux qui comportent des fonction isocyanurate, et dont le nombre de motifs monomère est inférieur à 5,
   (iii) des composés porteurs d'au moins une fonction isocyanurate et d'au moins une fonction urétidinedione, présentant une masse moléculaire supérieure à trois fois la masse moléculaire la plus élevée des composés isocyanates monomères ci-dessus,
   ledit mélange présentant un rapport fonctions carbonyles appartenant à un cycle urétidinedione/fonctions carbonyles appartenant à un cycle isocyanurate + fonctions carbonyles appartenant à un cycle urétidinedione au moins égal à 4 % ;
d) de 0 à 25 % en masse par rapport à la masse des composants a), b), c), d) et e) de composés porteurs d'au moins une fonction isocyanate, différents de a), b) et c) ; et
e) de 0 à 10 % en masse par rapport à la masse des composants a), b), c), d) et e) d'impuretés.

Par fonctionnalité moyenne élevée, on entend une fonctionnalité supérieure à 3, avantageusement supérieure à 3,5, de préférence supérieure à 4.

Il doit être compris qu'il n'existe pas de recouvrement entre les catégories a) à e) définies ci-dessus, et pas de recouvrement entre les catégories (i), (ii) et (iii) définies ci-dessus sous c). Ceci signifie que chacun des composants de la composition polyisocyanate appartient à une et une seule des catégories définies sous a), b), c)(i), c)(ii), c)(iii), d) et e). Autrement dit, chaque composant de la composition polyisocyanate selon la présente invention n'est comptabilisé qu'une seule fois dans les calculs des pourcentages des diverses catégories de composants formant ladite composition polyisocyanate.

L'expression "masse moléculaire moyenne des monomères isocyanates ayant la masse moléculaire la plus élevée (ou la plus petite)" signifie que lorsque un seul monomère est présent, ladite "masse moléculaire moyenne" est égale à celle dudit seul monomère, lorsque deux monomères distincts sont présents, ladite "masse moléculaire moyenne" est égale à celle du monomère ayant la masse moléculaire la plus élevée (la plus petite respectivement), et que lorsque trois monomères distincts, ou plus, sont présents, ladite "masse moléculaire moyenne" est égale à la masse moléculaire moyenne des deux monomères isocyanates ayant la masse moléculaire la plus élevée (ou la plus petite respectivement). En règle générale, pas plus de trois monomères distincts sont utilisés dans le cadre de la présente invention.

Par "viscosité réduite" au sens de la présente invention, on entend que la viscosité, mesurée à 25°C, exprimée en mPa.s, est réduite d'au moins 10 %, avantageusement au moins 12 %, de préférence au moins 20 %, par rapport à une composition polyisocyanate connue obtenue par cyclotrimérisation de monomères de départ identiques et présentant la même fonctionnalité.

D'une manière générale, la composition polyisocyanate selon la présente invention présente une viscosité comprise entre environ 1 000 mPa.s et environ 50 000 mPa.s. pour un taux de transformation supérieur à 35 %, de préférence supérieur à 40 %. Par exemple, dans le cas où le monomère de départ est l'HDI, la viscosité d'une composition selon l'invention est généralement inférieure à 25 000 mPa.s, avantageusement inférieure à 20 000 mPa.s pour un taux de transformation de 37 % des fonctions isocyanates, mesuré par dosage à la dibutylamine.

Le composant a) comprend les "composés mono-urétidinediones" qui sont le produit de condensation de deux molécules isocyanates monomères, également dénommés "dimères vrais" et représente avantageusement de 1 à 30 % en masse par rapport à la masse des composants a) + b) + c).

Le composant b) comprend les "composés mono-isocyanurates" qui sont le produit de condensation de trois molécules isocyanates monomères, également dénommés "trimères vrais", et représente de 5 à 40 % en masse par rapport à la masse des composants a) + b) + c).

Dans le mélange de composés c), les composés (i) et (iii) ont en commun le fait qu'ils sont compris dans la catégorie des "lourds" porteurs de fonctions isocyanurate. La catégorie b) comprend les composés porteurs d'une seule fonction isocyanurate. Dans la présente invention, on préfère en outre les compositions polyisocyanates dont le rapport en masse [c)(i) + c)(iii)]/b)] soit supérieur à 2, avantageusement supérieur à 3, de préférence supérieur à 4. Il a en effet été découvert de manière surprenante que les valeurs du ratio défini ci-dessus sont suffisantes pour obtenir les fonctionnalités élevées au sens de la présente invention.

Le mélange de composés c) comporte avantageusement :
- des composés polyisocyanates présentant deux cycles urétidinediones reliés par une chaîne hydrocarbonée ou plus ;
- des composés polyisocyanates présentant deux cycles isocyanurates reliés par une chaîne hydrocarbonée, ou plus.

Les composés comprenant au moins un cycle urétidinedione et au moins un cycle isocyanurate faisant partie du mélange c) comprennent avantageusement un groupe choisi parmi les formules (I) à (V) suivantes et leurs mélanges, 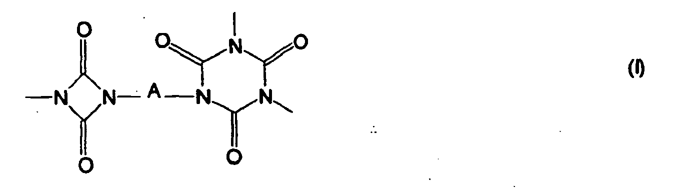 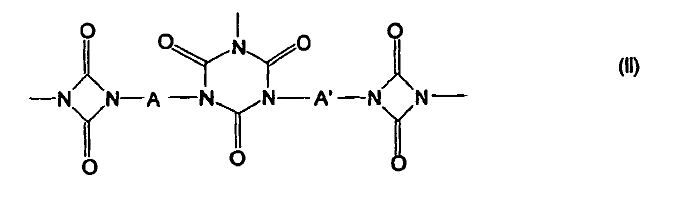 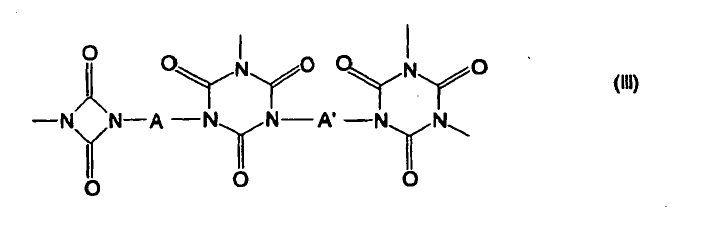 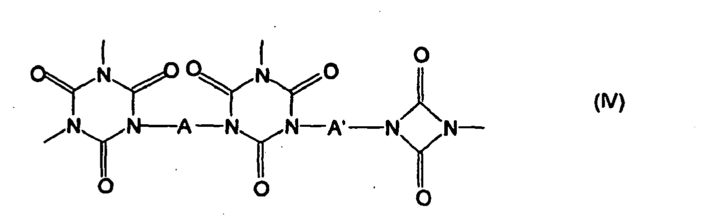 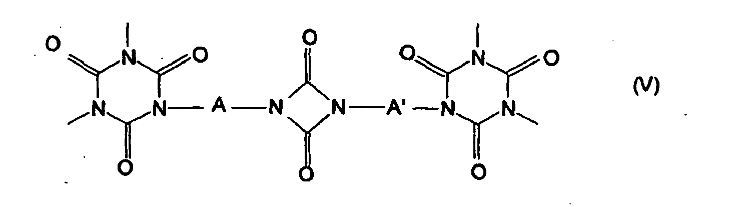 dans lesquels A et A' identiques ou différents représentent les restes d'un composé isocyanate monomère après enlèvement de deux fonctions isocyanates.

Avantageusement, A et A' identiques représentent une chaîne hydrocarbonée divalente comprenant des atomes de carbone et d'hydrogène exclusivement.

On préfère que les composés répondant à la catégorie c) représentent au moins 45 % avantageusement au moins 50 % en masse de la masse des composants a) + b) + c). En effet, il est bon de rappeler ici que plus la masse de composés classés sous c) est importante, plus le nombre de fonctions isocyanate (c'est-à-dire la fonctionnalité) augmente, sous réserve que le nombre de composés classés sous c) (ii) soit minoritaire par rapport aux autres composés classés sous c). Pour obtenir une fonctionnalité la plus élevée, il est préférable que la quantité de composés c) (ii) soit inférieure à 30% en poids par rapport à la totalité des composés classé sous c), de préférence inférieure à 20%, de préférence encore inférieure à 15%.

L'augmentation de la fonctionnalité s'obtient le plus souvent, comme dans le cas dans la présente invention, en augmentant le taux de transformation. Cependant, et comme cela ressort de l'art antérieur, ceci conduit généralement à une augmentation très sensible de la viscosité. Ce phénomène est moins observé avec les compositions de la présente invention.

II est en outre utile de rappeler ici que la formation d'un composé dit "lourd" à motif isocyanurate augmente la fonctionnalité moyenne de la composition de 1. Dans toute la présente description, la fonctionnalité est exprimée en masse, comme il est d'usage dans ce domaine, c'est-à-dire que l'on pondère la fonction de chaque élément au pourcentage de sa proportion massique.

Comme il a été dit auparavant, les compositions de la présente invention possèdent une fonctionnalité élevée, c'est-à-dire une fonctionnalité moyenne supérieure à 3, avantageusement supérieure à 3,5, de préférence supérieure à 4, sans toutefois présenter un viscosité aussi grande que celle décrite dans l'art antérieur pour des compositions polyisocyanates ayant une telle fonctionnalité.

Selon un mode de réalisation avantageux de l'invention, le composant d) représente au plus 10 % en masse de la masse totale des composants a) + b) + c) + d) + e), et représente au plus 5 % en masse de la masse des composants a) + b) + c) + d) + e).

Selon un mode de réalisation avantageux de l'invention, le composant e) représente de 0,05 % à 10 %, généralement de 0,1 % à 8 %, notamment au plus 5 % en masse par rapport à la masse totale des composants a) + b) + c) + d) + e) bien entendu les constituant de e) sont inertes vis à vis des fonctions isocyanates présentes en a), b), c) ou d).

Le composant e) consiste, de manière générale, en composés non porteurs de fonctions isocyanates libres, et notamment consiste en résidus de catalyseurs de polycondensation et/ou en sous-produits de polycondensation des monomères isocyanates de départ et/ou en solvant(s).

Le composant d) comprend ledit ou lesdits monomères isocyanates de départ qui représentent de 0,05 % à 20 %, plus généralement de 0,1 % à 10 %, avantageusement au plus 2 %, de préférence au plus 1 % en masse de la masse totale des composants a) + b) + c) + d) + e).

La composition d) comprend également des composés isocyanates avantageusement monomères, éventuellement ajoutés après la réaction de polycondensation des monomères diisocyanates, tels que des isocyanates ou triisocyanates de faible poids moléculaire (au plus 500), par exemple un lysine triisocyanate.

L'invention a également pour objet une composition telle que définie précédemment comportant en outre une quantité d'au plus 200 %, avantageusement au plus 100 % de préférence au plus 50 % en masse par rapport aux composants a), b), c), d) et e) d'un solvant organique ou mélange de solvants organiques.

Le solvant ou le mélange de solvants organiques est généralement liquide à température ambiante, ne comporte pas de fonction isocyanate, et ne comporte pas de fonction susceptible de réagir avec la fonction isocyanate, a un point d'ébullition d'au plus 300°C, avantageusement 250°C, de préférence au plus 200°C et est miscible avec les composants a), b), c) et d). Il est également avisé que le point de fusion dudit solvant (par solvant, on entend aussi les mélanges de solvant) soit au plus égal à l'ambiante, avantageusement à 0°C ; dans le cas des mélanges, les points de fusion ne sont pas francs (à l'exception bien entendu des eutectiques) et dans ce cas les valeurs ci-dessus se réfèrent au point de fusion finissante.

L'invention a également pour objet une composition polyisocyanate telle que définie ci-dessus comprenant de 1 à 100 %, avantageusement de 10 à 100 % des groupes NCO présents dans la composition ayant réagi avec un composé à hydrogène mobile. Certains des composés à hydrogène mobile sont appelés « agents masquants » dans la mesure où ils peuvent conduire à la restauration de la fonction isocyanate par un processus thermique ou physico-chimique. En général ces agents masquants restaurent la fonction isocyanate entre 50 et 200°C pendant un temps compris entre 5 minutes et une heure.

De façon préférée, les composés polyisocyanates de la composition de l'invention sont le produit de condensation de deux, trois ou plus de trois molécules isocyanates, porteurs de deux ou trois fonctions isocyanates, désignés dans la présente description par diisocyanates ou triisocyanates monomères.

II peut s'agir de monomères isocyanates à squelette hydrocarboné exclusivement de nature aliphatique, linéaire, ramifiés ou cycliques ou d'isocyanates aromatiques.

On peut citer en particulier comme monomère aliphatique linéaire l'hexaméthylènediisocyanate (HDI). On peut citer également les monomères aliphatiques dont le squelette hydrocarboné est ramifié mais dont les fonctions isocyanates sont portées par des atomes de carbone primaire, par exemple le 2-méthylpentane diisocyanate. On peut encore citer les monomères dont au moins une fonction isocyanate est en position cycloaliphatique, secondaire, tertiaire ou néopentylique.

Il s'agit notamment des monomères dans lesquels la fonction isocyanate est portée par un atome de carbone cycloaliphatique, secondaire, tertiaire ou néopentylique, en particulier les isocyanates cycloaliphatiques. Ces monomères sont tels qu'au moins l'une avantageusement des deux fonctions isocyanates est distante du cycle le plus proche d'au plus un carbone et, de préférence, est reliée directement à lui. En outre, ces monomères cycloaliphatiques présentent avantageusement au moins une, de préférence deux fonctions isocyanates choisies parmi les fonctions isocyanates secondaire, tertiaire ou néopentylique.

On peut citer à titre d'exemple les monomères suivants :
- les composés correspondant à l'hydrogénation du ou des noyaux aromatiques porteurs des fonctions isocyanates de monomères d'isocyanates aromatiques et notamment du TDI (toluènediisocyanate) et des diisocyanatobiphényles, le composé connu sous le sigle H₁₂MDI (4,4' bis-(isocyanatocyclohexyl)méthane), les divers BIC [Bis(isocyanatométhylcyclohexane)] et les cyclohexyldiisocyanates éventuellement substitués ; et surtout
- le norbomanediisocyanate, souvent désigné par son sigle NBDI ;
- l'isophoronediisocyanate, ou IPDI, ou plus précisément 3-isocyanatométhyl-3,5,5-triméthylcyclohexylisocyanate.
   Comme monomères aromatiques, on peut citer les :
   - 2,4- ou 2,6- toluène diisocyanate (TDI) ;
   - 2,6-(4,4'-diphénylméthane)diisocyanate (MDI) ;
   - 1,5-naphtalène diisocyanate (NDI) ;
   - para-phénylène diisocyanate (PPDI).

On préfère les monomères isocyanates aliphatiques y compris cycloaliphatiques, les préférés étant les aliphatiques comportant des enchaînements polyméthylène. Par monomère isocyanate aliphatique, on entend des monomères dont au moins une fonction isocyanate est rattachée à un atome de carbone d'hybridation sp³, avantageusement deux fonctions isocyanates, de préférence toutes les fonctions isocyanates sont rattachées à des atomes de carbone d'hybridation sp³.

Les monomères isocyanates de départ de faible masse moléculaire tels que définis ci-dessus ont généralement une teneur en groupes isocyanates d'au moins 12 %, de préférence au moins 15 %, de préférence au moins 20 %, exprimé en poids de NCO par rapport au poids total d'isocyanate.

Les monomères de départ peuvent également être des produits d'oligomérisation d'isocyanates de faible masse moléculaire tels que définis ci-dessus, ces produits d'oligomérisation étant porteurs de fonctions isocyanates masquées ou non.

Le groupe masquant est la résultante de la réaction d'un composé ayant au moins un atome d'hydrogène réactif avec la fonction isocyanate des polyisocyanates tels que définis ci-dessus.

L'agent masquant, qui peut être un mélange d'agents masquants, est tel que la réaction de masquage puisse s'écrire :

Is-N=C=O + AM-H → Is-NH-CO(AM)

où AM-H représente l'agent masquant ;
où AM- représente le groupe masquant ;
où Is est le reste porteur de la fonction isocyanate considérée.

Ledit agent masquant présente au moins une fonction portant un hydrogène mobile, ou plus exactement réactif, fonction pour laquelle on peut définir un pKa lequel correspond, soit à l'ionisation d'un acide, y compris l'hydrogène des fonctions, phénols et alcools, soit à l'acide associé d'une base, en général azotée. Le pKa de la fonction présentant des hydrogènes est au moins égal à 4, avantageusement à 5, de préférence à 6 et est au plus égal à 14, avantageusement à 13, de préférence à 12, et de manière plus préférée à 10, une exception devant être faite pour les lactames dont le pKa est supérieur à ces valeurs et qui constituent des agents masquants néanmoins acceptables bien que non préférés pour l'invention.

Avantageusement, l'agent masquant ne comporte qu'un seul hydrogène mobile.

A titre d'exemples non limitatifs, des agents de masquage selon l'invention, on peut citer les dérivés de l'hydroxylamine tels que l'hydroxysuccinimide et les oximes telles tel que la méthyléthylcétoxime ou le pyruvate oxime de méthyle, les dérivés des phénols ou assimilés, les dérivés des amides tels les imides et les lactames, ainsi que les malonates ou cétoesters et les hydroxamates.

On peut également citer les groupes hétérocycliques azotés comprenant 2 à 9 atomes de carbone et, outre l'atome d'azote, de 1 à 3 autres hétéroatomes choisis parmi l'azote, l'oxygène et le soufre. Ces groupes sont par exemple choisis parmi les groupes pyrolyle, 2H-pyrrolyle, imidazolyle, pyrimidinyle, pyridazinyle, pyrazinyle, pyrimidinyle, pyridazinyle, indolizinyle, iso-indolyle, indolyle, indozolyle, purinyle, quinolizinyle, isoquinolyle, pyrazolidinyle, imidazolidinyle et triazolyle. On préfère en particulier les hétérocycles comportant de 2 à 4 atomes de carbone et de 1 à 3 atomes d'azote, tels les groupes pyrazolyle, imidazolyle et triazolyle, ces groupes étant éventuellement substitués par un à trois substituants choisis parmi NH₂, NH(alkyle en C₁-C₆), N-(dialkyle en C₁-C₆), OH, SH, CF₃, alkyle en C₁-C₆, cycloalkyle en C₃-C₆, aryle en C₅-C₁₂, notamment phényle, aralkyle en C₆-d₁₈ ayant de 5 à 12 atomes de carbone dans le groupe aryle, notamment benzyle ou alkaryle en C₆-C₁₈ ayant de 5 à 12 atomes de carbone dans le groupe aryle.

Particulièrement préférés sont les groupes 1,2,3-triazolyle ou 1,2,4-triazolyle, ou 3,5-diméthyl-pyrazolyle.

Pour la détermination des pKa, on pourra se reporter à "The détermination of ionization constants, a laboratory manual, A. Albert of E.P. Serjeant ; Chapman and Hall Ltd, London".

Pour la liste des agents masquants, on pourra se reporter à Z. Wicks (Prog. Org. Chem., (1975), 3, 73 et Prog. Org. Chem., (1989), 9, 7) et Petersen (Justus Liebigs, Anna/en der Chemie, 562, 205, (1949).

Le solvant organique est avantageusement choisi parmi :
- les hydrocarbures aromatiques, notamment le toluène, le xylène, le Solvesso® ;
- les esters, comme l'acétate de n-butyle, l'adipate de diméthyle, le glutarate de méthyle, ou leurs mélanges ;
- les esters éthers comme l'acétate de méthoxypropyle ;
- les éthers comme le butylglycol éther ;
- les cétones, comme la méthyl-isobutyl cétone ;
- les solvants fluorés comme le trifluorométhylbenzène.

La composition polyisocyanate selon l'invention peut être obtenue par un procédé comprenant les étapes suivantes :
i) on prépare un milieu réactionnel de départ comprenant le(s) isocyanate(s) monomère(s) de départ, et éventuellement d'autres monomères réactifs avec la fonction isocyanate ;
ii) on fait réagir le milieu réactionnel de départ en présence d'un catalyseur de dimérisation, éventuellement en chauffant le milieu réactionnel à une température d'au moins 40°C ;
iii) on fait réagir le produit réactionnel de l'étape ii) contenant des monomères n'ayant pas réagi avec un catalyseur de (cyclo)trimérisation, dans les conditions de (cyclo)trimérisation ;
iv) on élimine du produit réactionnel de l'étape iii) les monomères de départ n'ayant pas réagi ;
ledit procédé étant caractérisé en ce que l'on effectue l'étape iii) jusqu'à un taux de transformation d'au moins 35 %, avantageusement d'au moins 40 % des monomères isocyanates présents dans le milieu réactionnel initial.

Le taux de monomères résiduel est mesuré par dosage après séparation du mélange polyisocyanate sur une colonne séparative de type chromatographie de filtration sur gel dans un solvant tel que dichlorométhane (CH₂Cl₂) ou tétrahydrofurane (THF). La méthode de détection est l'infrarouge en mesurant la bande NCO à 2 250 cm⁻¹, après étalonnage avec un monomère de concentration connue.

En variante, la composition polyisocyanate selon l'invention peut également être obtenue par un procédé comprenant les étapes suivantes :
i) on prépare un milieu réactionnel de départ comprenant le(s) isocyanate(s) monomère(s) de départ, et éventuellement d'autres monomères réactifs avec la fonction isocyanate ;
ii) on fait réagir les monomères de départ, avec un catalyseur de (cyclo)trimérisation dans les conditions de (cyclo)trimérisation ;
iii) on fait réagir le milieu réactionnel de l'étape ii) en présence d'un catalyseur de dimérisation, éventuellement en chauffant le milieu réactionnel à une température d'au moins 40°C ;
iv) on élimine du produit réactionnel de l'étape iii) les monomères de départ n'ayant pas réagi ;
   ledit procédé étant caractérisé en ce que l'on effectue l'étape iii) jusqu'à un taux de transformation d'au moins 35 %, avantageusement d'au moins 40 % des monomères isocyanates présentes dans le milieu réactionnel initial.

La réaction peut être effectuée en l'absence ou en présence d'un solvant. On préfère généralement le réaliser en l'absence de solvant.

On peut également, conformément au procédé de l'invention, préparer la composition polyisocyanate de manière continue, en soutirant les monomères de départ n'ayant pas réagi et en les recyclant vers l'étape de polycondensation.

Les composés a), b) et c) selon l'invention peuvent être obtenus à partir d'un seul monomère ou mélange de monomères différents.

La réaction de formation des composés urétidinediones peut être réalisée par voie catalytique exclusivement, en l'absence de chauffage. Cette dimérisation catalytique en l'absence chauffage est un des modes avantageux selon la présente invention. Ces catalyseurs spécifiques sont ceux qui sont connus de l'homme du métier pour donner des urétidinediones à partir de fonctions isocyanates, et ce, avec peu de, ou aucun autre type de condensation (notamment formation d'isocyanurate). Quelques exemples paradigmatiques de tels catalyseurs spécifiques de la dimérisation sont donnés ci après.

Ainsi, les catalyseurs spécifiques de dimérisation sont ceux qui sont connus de l'homme du métier pour former des groupements urétidinediones à partir de fonctions isocyanates et sont choisis parmi les composés de type tris-(N,N-dialkyl)phosphotriamide, N,N-dialkylaminopyridine ou encore de type trialkylphosphine. Un exemple tout particulièrement préféré de catalyseurs de dimérisation pour l'obtention des compositions selon l'invention est représenté par les catalyseurs de type trialkylphosphine.

Selon une variante, il peut être avantageux de chauffer le milieu réactionnel lors de la réaction de formation des composés urétidinediones par voie catalytique. La réaction de dimérisation est alors effectuée par voie catalytique et thermique. Il est à noter également qu'avec certains catalyseurs, tels que les trialkylphosphines, l'augmentation de la température favorise la réaction de trimérisation.

Certains catalyseurs, tels ceux de type tris-(N,N-dialkyl)phosphotriamide et N,N-dialkylaminopyridine, conduisent très majoritairement à la formation spécifique de dimères. Lorsque de tels catalyseurs dits de dimérisation sont utilisés, il peut être avantageux d'ajouter un catalyseur de trimérisation de manière à réaliser conjointement les réactions de dimérisation et de trimérisation.

En outre, certains catalyseurs permettent à la fois les réactions de dimérisation et de trimérisation. Un exemple de ces catalyseurs est représenté par ceux de type trialkylphosphine.

L'originalité du procédé catalytique de dimérisation dans la présente invention réside dans le fait que, bien que les réactions de dimérisation catalytiques soient connues en soi, cette dimérisation catalytique est conduite jusqu'à obtenir des taux de transformation des espèces monomères et/ou oligomères de départ supérieurs à 35 %, avantageusement supérieurs à 40 %, voir encore supérieurs à ces limites.

Cette formation de cycles urétidinediones est notamment réalisée en présence de composés de type trialkylphosphine, tris-(N,N-dialkyl)phosphotriamide, N,N,N',N'-tétra-alkylguanidines.

Le catalyseur de cyclotrimérisation peut être tout catalyseur connu à cet effet. On peut citer les amines tertiaires telles que la triéthylamine, les bases de Mannich telles que le tris-(N,N-diméthylaminométhyl)phénol, les hydroxydes ou sels d'acides organiques faibles ou fluorures de tétra-alkylammoniums tels que les tétraméthyl-, tétra-éthyl- et tétra-butyl-ammoniums, les hydroxydes et sels d'acides organiques faibles d'hydroxy-alkylammoniums tels que le N,N,N-triméthyl-N-hydroxyéthyl-ammonium carboxylate ou le N,N,N-triméthyl-N-hydroxypropylammonium hydroxyde ; les sels de métaux alcalins, alcalino-terreux, d'étain, de zinc
ou d'autres métaux d'acides carboxyliques, tels que l'acide acétique propionique, octanoïque, ou benzoïque ou les carbonates de ces métaux ; les alcoolates ou phénates de métaux alcalins, alcalino-terreux, étain, zinc ou autres métaux ; les alkyl phosphines tertiaires telles que décrites dans US 3,211,703, les composés de métaux lourds tels que l'acétylacétonate de fer décrits dans US 3,135,111, les amines silylées et hexaméthyldisilazane décrites dans EP 89297 ; ou les alcoolates de terres rares décrits dans FR 99 16 687.

À l'issue de la réaction de cyclotrimérisation, on désactive le catalyseur de cyclotrimérisation par tout moyen connu, notamment par addition d'un poison de catalyseur ou encore par absorption sur une colonne d'alumine.

Dans le cas où on effectue l'étape de cyclodimérisation catalytique après la réaction de cyclotrimérisation, il conviendra de s'assurer que le catalyseur de trimérisation est effectivement désactivé.

D'une manière générale, lorsqu'un catalyseur de dimérisation et/ou de trimérisation est employé dans les procédés tels qu'ils viennent d'être décrits, il doit être compris que les compositions polyisocyanates selon la présente invention peuvent contenir en outre des traces de ces catalyseurs ou de leurs dérivés issus de leur décomposition.

Lorsque l'on souhaite obtenir une composition polyisocyanate masquée telle que définie ci-dessus, on fait réagir les fonctions isocyanates présentes dans le milieu réactionnel avec l'agent masquant avant, pendant ou après les étapes décrites ci-dessus.

On préfère toutefois réaliser la réaction de masquage après la réaction de polycondensation et après élimination des isocyanates monomères n'ayant pas réagi.

L'un des nombreux intérêts des compositions polyisocyanates selon l'invention est qu'elles peuvent servir de base à la préparation de polymères et/ou de réticulats et être utilisées notamment comme un des constituants principaux de revêtements en tous genres, tels que vernis et peintures. Dans de telles utilisations, les qualités de dureté des polymères réticulables font partie de celles qui sont recherchées sur le plan technique et fonctionnel.

Le procédé de préparation de polymères comporte les étapes suivantes :
- mettre une composition polyisocyanate selon l'invention en présence d'un co-réactif qui contient des dérivés présentant des hydrogènes réactifs sous forme d'alcool, de phénol, de thiol, de certaines amines y compris les anilines ; ces dérivés peuvent avoir des squelettes hydrocarbonés aliphatiques, alicycliques
ou aromatiques, de préférence alcoyles, y compris cycloalcoyles et aralcoyles, aryles, linéaires ou branchés, substitués ou non (ces co-réactifs, en général des polyols sont en eux-mêmes connus) et forment après réaction avec les polyisocyanates un réseau ;
- et chauffer le milieu réactionnel ainsi constitué à une température permettant la réticulation des composants.

Avantageusement, la température est au plus égale à environ 300°C, de préférence au moins égale à 60°C, plus préférentiellement au moins égale à 80°C et de préférence au plus égale à 250°C et plus préférentiellement encore à 200°C et ce, pour une durée inférieure ou égale à 15 heures, de préférence à 10 heures, et plus préférentiellement encore à 8 heures. Il est connu de l'homme de métier que plus la température est élevée moins il faut de temps pour réaliser la réticulation par cuisson. Ainsi une cuisson à 300°C ne nécessite que quelques dizaines de secondes voire quelques minutes, alors que qu'une température de 60°C nécessite un temps qui s'exprime en heures.

On peut prévoir d'inclure un solvant organique dans le milieu réactionnel de réticulation. On peut également prévoir une suspension dans l'eau.

Ce solvant optionnel est, de préférence peu polaire, c'est-à-dire dont la constante diélectrique n'est guère supérieure ou égale à 4 ou plus préférentiellement à 5.

Conformément à l'invention, les solvants peu polaires préférés sont ceux qui sont bien connus de l'homme du métier et en particulier les aromatiques tel que le benzène, les cétones telles que la cyclohexanone, la méthyléthylcétone et l'acétone; les esters d'alcoyle(s) légers et notamment les esters adipiques ; les coupes pétrolières du type dé celles vendues sous la marque Solvesso®.

Généralement, le solvant est identique au solvant de la composition polyisocyanate ci-dessus.

Les dérivés entrant dans la composition du co-réactif sont en général di-, oligo-, ou polyfonctionnels, peuvent être monomères ou issus de di-, d'oligo- ou poly-mérisation et sont mis en oeuvre pour la préparation de polyuréthannes éventuellement réticulés, leur choix sera dicté par les fonctionnalités attendues pour le polymère dans l'application finale et par leur réactivité.

Notamment lorsque l'on désire avoir des compositions "bi-composant" (c'est-à-dire contenant simultanément les deux réactifs : la composition polyisocyanate ici au moins partiellement masquée selon l'invention et le composé à hydrogène réactif) stables, on préfère éviter d'utiliser des dérivés présentant des hydrogènes réactifs qui catalysent la libération de l'isocyanate masqué. Ainsi, parmi les amines, on préfère n'utiliser que celles qui ne catalysent pas la décomposition
ou la transamidation des fonctions isocyanates masquées selon la présente invention. Ces co-réactifs sont en général bien connus de l'homme de métier.

L'invention concerne donc, également, des compositions de peintures comprenant pour addition successive ou simultanée :
- un polyisocyanate masqué selon l'invention ;
- un co-réactif à hydrogène réactif tel que décrit ci-dessus ;
- d'éventuels catalyseurs en eux-mêmes connus (notamment ceux à base d'étain pour les oximes) ;
- éventuellement au moins un pigment, tel que du bioxyde de titane ;
- éventuellement une phase aqueuse ;
- éventuellement un agent tensio-actif pour maintenir en émulsion ou en suspension les composants constitutifs du mélange ;
- éventuellement un solvant organique ;
- éventuellement un déshydratant.

Les catalyseurs sont avantageusement latents, notamment ceux qui ont fait l'objet de brevet et de demande de brevet publié au nom de la demanderesse ou de ses prédécesseurs en droit (société comportant dans son nom « Rhône-Poulenc »).

L'invention concerne aussi les peintures et vernis obtenues par l'utilisation de ces compositions, selon le procédé ci-dessus.

Les exemples ci-après illustrent l'invention.

### Abréviations utilisées :

HMDZ : hexaméthyldisilazane ;
HDI : hexaméthylenediisocyanate ;
AcO-n-butyle : Acétate de n-butyle ;
Fonction NCO : Fonction isocyanate
MEKO : Butanone ou Méthyléthylcétoxime
DMP : 3,5-Diméthylpyrazole
poly NCO : Polyisocyanate

Les quantités des différents composants présentés dans la présente description, exemples et revendications sont exprimées en pourcentage en masse, sauf indication contraire.

La signification des termes vernaculaires usuels est rappelée ci-dessous :
- Bis-Dimère : oligomère issu de la condensation de trois monomères et présentant deux motifs urétidinedione ;
- Tris-Dimère : tétramère à trois motifs urétidinedione ;
- Bis-Trimère : pentamère à deux motifs isocyanurate ;
- Trimère-Dimère : tétramère à un motif isocyanurate et à un motif urétidinedione ;
- Trimère-Dimère-Trimère : hexamère à deux motifs isocyanurate et à un motif urétidinedione.
- Les « lourds » correspondent aux oligomères ayant une masse moléculaire égale ou supérieure à 7 fois celle des monomères.

Rappelons que selon la technique usuelle en la matière, les différents composants de la fraction oligomériques sont identifiées par analyse structurales infra rouge, leurs répartitions et leurs fonctionnalités sont données et quantifiées en utilisant les bandes caractéristiques des composés polyisocyanates à savoir les bandes des fonctions isocyanates, les bandes alkyles, les bandes CO de l'isocyanurate et celles de l'urétidinedione. On obtient ainsi la répartition oligomérique pondérale correspondant à chaque synthèse exemplifiée.

À chaque oligomère et à chaque fraction oligomérique correspond une fonctionnalité mesurée (par la teneur en fonction NCO), qui dans la cas des oligomères purs peut être comparée avec la théorique (ainsi le dimère de HDI a une fonctionnalité de 2).

Rappelons que dans le domaine, la fonctionnalité est obtenue de la manière suivante : on multiplie le pourcentage pondéral de chaque oligomère de la composition par sa fonctionnalité propre puis on somme les fonctionnalités apportées par chaque oligomère. Le total représente la fonctionnalité moyenne de la composition oligomérique. Dans le cas de la présente invention, les compositions finales sont soumises à une séparation sur un ensemble de colonnes de perméation de gel vendues par la société Polymer Laboratories sous la marque PL Gel type mixte E.

Le titre NCO est mesuré, de manière habituelle, selon la norme AFNOR NF T 52-132 de septembre 1988 (parfois désigné par méthode à la dibutylamine).

La viscosité est mesurée, de manière usuelle, selon la norme NF EN ISO 3219 de novembre 1994 (Méthode de détermination de la viscosité par la méthode du cylindre tournant).

Les exemples 1 et 2 suivants sont représentatifs de polyisocyanates de faible viscosité et à haute fonctionnalité utilisant la tributylphosphine comme catalyseur.

### Exemple 1 :

Dans un réacteur tricol équipé d'une agitation mécanique, d'un réfrigérant et d'une ampoule de charge, on ajoute sous azote 310 g de HDI. On dégaze le milieu réactionnel par bullage d'azote pendant 1 heure, puis on ajoute 1,18 g de tri-n-butylphosphine sous azote. Le milieu réactionnel est chauffé à 60 °C environ pendant 10 heures.
Lorsque le taux de transformation de fonctions NCO mesuré selon la méthode à la dibutylamine est de 39,6 %, (ce qui correspond à un taux de transformation théorique en HDI de 79,2%), on bloque la réaction par addition de un équivalent molaire de tosylate de méthyle par rapport au catalyseur introduit. Ce taux de transformation de 39,6 % correspond à un taux de transformation en HDI de 63,1% mesuré sur le mélange réactionnel avant élimination du monomère HDI selon la technique de chromatographie par perméation de gel. On laisse le milieu réactionnel sous agitation pendant 2 heures à 80°C.
Le produit est purifié par distillation de l'HDI monomère non transformé, sous vide de 0,4 mbar, à 160°C, sur un appareil à film mince, avec un débit d'environ 200 g/heure. Cette opération est renouvelée une seconde fois pour donner 162 g de produit d'oligomérisation de l'HDI, soit un rendement récupéré de 52 %.

Les caractéristiques du produit sont les suivantes :
Taux de transformation : 63 %
Titre NCO du mélange réactionnel après distillation : 18,7 %
Viscosité du mélange réactionnel à 25°C : 1043 mPa.s.
Fonctionnalité : de l'ordre de 3,5.

La composition du mélange obtenu, après élimination du monomère HDI, est présentée dans le tableau ci après.

| **C*atégorie*** | ***Composé*** | ***Fonctionnalité*** | **% *en poids*** | ***Participation** à l**a fonctionnalité*** |
|---|---|---|---|---|
| | HDI (monomère de départ) | 2 | 1 | 0.02 |
| a) | Urétidinedione de HDI (Dimère de HDI) | 2 | 24 | 0,48 |
| b) | Trimère | 3 | 15 | 0,45 |
| c) (ii) | Bis Dimère | 2 | 8 | 0,16 |
| c) (iii) | Trimère-Dimère | 3 | 10 | 0,3 |
| c) (ii) | Tris Dimère | 2 | 4 | 0,08 |
| c) (i) et (iii) | Bis Trimère + (Trimère-Dimère-Trimère) | 4 | 12 | 0,48 |
| c) (i) et (iii) | Lourds | ∼ 5,8 | 26 | 1,51 |

- Le ratio dimère vrai sur la somme (dimère + trimère vrais ) est de 0,61.
- Le ratio C=O Dimère/C=O (Dimère + Trimère) mesuré sur la répartition globale est de 43%.
- Les composés lourds sont constitués d'enchaînements tels que définis dans l'invention à savoir des enchaînements de séquence Trimère-Dimère, Trimère-Trimère-Dimère, Trimère-Dimère-Trimère, etc.
- Le rapport des composés [(Trimère Dimère) + (Tris Dimère) + (BisTrimère) + (Trimère-Dimère-Trimère) + Lourds] sur les composés Trimère, c'est-à-dire le rapport [(c)(i) + c)(iii))/b)] est de l'ordre de 3,5.

### Exemple 2 :

On procède comme pour l'exemple précédent sauf que la quantité de tri-n-butylphosphine est de 2,25g et le taux de transformation en HDI mesuré après chromatographie de perméation de gel est de 85,6 %.

La fonctionnalité est de l'ordre de 5.

La répartition des espèces avant distillation du monomère est la suivante :

| ***Catégorie*** | ***Composé*** | ***Fonctionnalité*** | ***% en poids*** | ***Participation à la fonctionnallité*** |
|---|---|---|---|---|
| | HDI (monomère de départ) | 2 | 14,4 | ‡ |
| a) | Urétidinedione de HDI (Dimère de HDI) | 2 | 5 | 0,116 |
| b) | Trimère | 3 | 7 | 0,245 |
| c) (ii) | Bis Dimère | 2 | 1,2 | 0,028 |
| c) (iii) | Trimère-Dimère | 3 | 3,3 | 0,115 |
| c) (ii) | Tris Dimère | 2 | 1,1 | 0,025 |
| c) (i) et (iii) | Bis Trimère + (Trimère-Dimère-Trimère) | 4 | 6,3 | 0,294 |
| c) (i) et (iii) | Lourds | ∼ 6 | 61,7 | 4,32 |

| | | | | |
|---|---|---|---|---|
| ‡: non pris en compte dans le calcul de la fonctionnalité totale de la composition | | | | |

• Les composés lourds sont constitués d'enchaînements tels que définis dans l'invention à savoir des enchaînements de séquence Trimère-Dimère, Trimère-Trimère-Dimère, Trimère-Dimère-Trimère, etc.
• Le rapport des composés [(Trimère Dimère) + (Tris Dimère) + (BisTrimère) + (Trimère-Dimère-Trimère) + Lourds] sur les composés Trimère, c'est-à-dire le rapport [(c)(i) + c)(iii))/b)] est de l'ordre de 10.
• La répartition entre fonctions dimère et trimère est la suivante :

| | % fonctions C=O trimère | % fonctions C=O dimère | Ratio C=O Dimère / C=O (trimère + dimère) |
|---|---|---|---|
| Répartition globale | 69,1% | 30,9 % | 30,9 % |
| Lourds | 56,2 % | 21,7 % | 27,9 % |
| Espèces Bis * | 6,9 % | 4,2 % | 37,9 % |
| Espèces Vraies** | 6 % | 5 % | 45,3 % |

| | | | |
|---|---|---|---|
| * En tant qu'espèces Bis on entend le bis dimère et le bis trimère ** En tant qu'espèces vraies on entend le dimère (composant a)) et le cyclotrimère (composant b)). | | | |

## Revendications

1. Composition polyisocyanate ayant une fonctionnalité moyenne supérieure à 3, avantageusement supérieure à 3,5, de préférence supérienre à 4, obtenue par polycondensation de monomères diisocyanates ou triisocyanates, comportant :
(a) de 0,5 à 30 % en masse par rapport à la masse totale des composants a), b) et c) de composés porteurs d'une seule fonction urétidinedione ayant une masse moléculaire au plus égale à deux fois la masse moléculaire moyenne des monomères isocyanates ayant la masse moléculaire la plus élevée;
(b) de 0,5 à 45 % en masse par rapport à la masse totale des composants a), b) et c) de composés porteurs d'une seule fonction isocyanurate et de masse moléculaire au plus égale à trois fois la masse moléculaire moyenne desdits monomères isocyanates ayant la masse moléculaire la plus élevée ; le rapport molaire de (a)/(b) étant compris entre 0,02 et 2, avantageusement entre 0,2 et 1,8, de préférence inférieur ou égal à 1,6,
(c) au moins 40 % en masse par rapport à la masse totale des composants a), b) et c) d'un mélange de composés polyisocyanates présentant une masse moléculaire au moins égale à trois fois la masse moléculaire moyenne des monomères isocyanates ayant la masse moléculaire la plus petite et porteurs d'au moins deux fonctions isocyanates, et
ledit mélange comportant :
(i) des composés porteurs d'au moins deux fonctions isocyanurates, à l'exclusion de ceux qui comportent des fonctions urétidinedione,
(ii) des composés porteurs d'au moins deux fonctions urétidinediones, à l'exclusion de ceux qui comportent des fonctions isocyanurate, et dont le nombre de motifs monomère est inférieur à 5,
(iii) des composés porteurs d'au moins une fonction isocyanurate et d'au moins une fonction urétidinedione, présentant une masse moléculaire supérieure à trois fois la masse moléculaire la plus élevée des composés isocyanates monomères ci-dessus ; ledit mélange présentant un rapport fonctions carbonyles appartenant à un cycle urétidinedione/fonctions carbonyles appartenant à un cycle isocyanurate + fonctions carbonyles appartenant à un cycle urétidinedione au moins égal à 4 % ;
d) de 0 à 25 % en masse par rapport à la masse des composants a), b), c), d) et e) de composés porteurs d'au moins une fonction isocyanate, différents de a), b) et c) ; et
e) de 0 à 10 % en masse par rapport à la masse des composants a), b), c), d) et e) d'impuretés.

2. Composition polyisocyanate selon la revendication 1, **caractérisée en ce qu'**elle comporte de 1 à 30% en masse du composant (a) par rapport à la masse totale des composants a) + b) + c).

3. Composition polyisocyanate selon la revendication 1, **caractérisée en ce qu'**elle comporte de 5 à 40 % en masse du composant (b) par rapport à la masse totale des composants a) + b) + c).

4. Composition polyisocyanate selon la revendication 1, **caractérisée en ce que** le composant c) représente au moins 45 %, avantageusement au moins 50 % en masse par rapport à la masse totale des composants a) + b) + c).

5. Composition polyisocyanate selon la revendication 1, **caractérisée en ce que** le rapport en masse [c)(i) + c)(iii)]/b) soit supérieur à 2, avantageusement supérieur à 3, de préférence supérieur à 4.

6. Composition polyisocyanate selon la revendication 1, **caractérisée en ce que** la quantité de composés c) (ii) est inférieure à 30% en poids par rapport à la totalité des composés classé sous c), de préférence inférieure à 20%, de préférence encore inférieure à 15%.

7. Composition polyisocyanate selon la revendication 1, **caractérisée en ce que** le composant d) représente au plus 10 % en masse par rapport à la masse totale des composants a) + b) + c) + d) + e).

8. Composition polyisocyanate selon la revendication 1, **caractérisée en ce que** le composant e) représente de 0,05 % à 10 %, généralement de 0,1 % à 8 %, notamment au plus 5 % en masse par rapport à la masse totale des composants a) + b) + c) + d) + e).

9. Composition polyisocyanate selon la revendication 1, **caractérisée en ce que** le composant e) consiste en résidus de catalyseur de polycondensation et/ou en sous-produits de polycondensation des monomères isocyanates de départ et/ou en solvant(s).

10. Composition polyisocyanate selon la revendication 1, **caractérisée en ce que** le composant d) comprend ledit ou lesdits monomères isocyanates résiduels.

11. Composition polyisocyanate selon la revendication 10, **caractérisée en ce que** ledit ou lesdits monomères isocyanates représentent de 0,05 % à 20 %, plus généralement de 0,1 % à 10 %, avantageusement au plus 2 %, de préférence au plus 1 %, en masse de la masse des composants a) + b) + c) + d) + e).

12. Composition selon l'une quelconque des revendications précédentes, **caractérisée en ce qu'**elle comporte en outre une quantité d'au plus 200 %, avantageusement au plus 100 % en masse de a) + b) + c) + d) + e), de préférence au plus 50 % un solvant organique ou mélange de solvants organiques liquide à température ambiante, ne comportant pas de fonction isocyanate, ne comportant pas de fonction susceptible de réagir avec la fonction isocyanate, ayant un point d'ébullition d'au plus 200 °C et étant miscible avec les composants a), b), c), d) et e).

13. Composition selon l'une quelconque des revendications 1 à 12, **caractérisée en ce que** les composés comportant au moins un cycle urétidinedione et au moins un cycle isocyanurate comprennent un groupe choisi parmi les formules (I) à (V) suivantes et leurs mélanges : 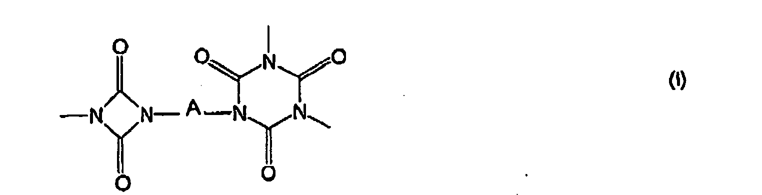 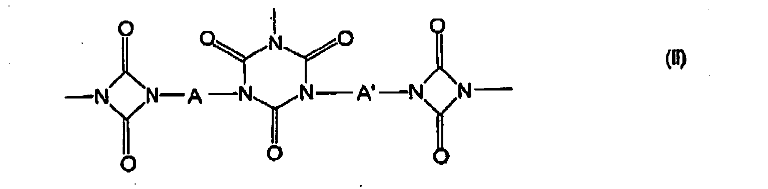 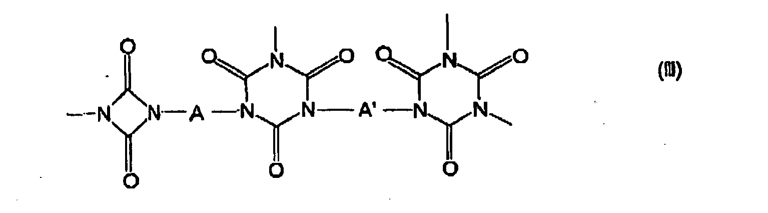 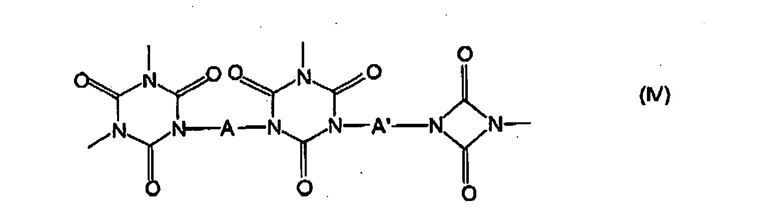 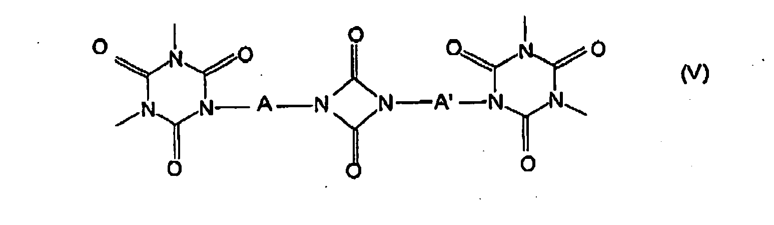 dans lesquels A et A' identiques ou différents représentent les restes d'un composé isocyanate monomère après enlèvement de deux fonctions isocyanates.

14. Composition selon l'une quelconque des revendications précédentes, **caractérisée en ce qu'**elle comporte de 1 à 100 %, avantageusement de 10 à 100 % des groupes NCO présents dans la composition masqués à l'aide d'un agent masquant.

15. Composition selon la revendication 14, **caractérisée en ce que** l'agent masquant est un agent masquant monofonctionnel choisi parmi les dérivés de l'hydroxylamine, les oximes, les dérivés des phénols, les dérivés des amides, les maionates, les cétoesters, les hydroxamates et les composés hétérocycliques azotés.

16. Composition selon la revendication 15, **caractérisée en ce que** l'agent masquant est la méthyléthylcétoxime ou le pyruvate oxime de méthyle.

17. Composition selon la revendication 14, **caractérisée en ce que** l'agent masquant est choisi parmi les groupes pyrolyle, 2H-pyrrolyle, imidazolyle, pyrimidinyle, pyridazinyle, pyrazinyle, indolizinyle, iso-indolyle, indolyle, indozolyle, purinyle, quinolizinyle, isoquinolyle, pyrazolidinyle, imidazolidinyle et triazolyle.

18. Procédé de réparation d'une composition polyisocyanate selon l'une des revendications 1 à 17 comprenant les étapes suivantes :
i) on prépare un milieu réactionnel de départ comprenant le(s) isocyanate(s) monomère(s) de départ, et éventuellement d'autres monomères réactifs avec la fonction isocyanate ;
ii) on fait réagir le milieu réactionnel de départ en présence d'un catalyseur de dimérisation, éventuellement en chauffant le milieu réactionnel à une température d'au moins 40°C ;
iii) on fait réagir le produit réactionnel de l'étape ii) contenant des monomères n'ayant pas réagi avec un catalyseur de (cyclo)trimérisation, dans les conditions de (cyclo)trimérisation ;
iv) on élimine du produit réactionnel de l'étape iii) les monomères de départ n'ayant pas réagi ;
v) éventuellement, on fait réagir le milieu réactionnel avec un agent masquant avant, pendant, ou après les étapes i) à iv) ;
ledit procédé étant **caractérisé en ce que** l'on effectue l'étape iii) jusqu'à un taux de transformation d'au moins 35 %, avantageusement d'au moins 40 % des monomères isocyanates présents dans le milieu réactionnel initial.

19. Procédé de préparation d'une composition polyisocyanate selon l'une quelconque des revendications 1 à 18 comprenant les étapes suivantes :
i) on prépare un milieu réactionnel de départ comprenant le(s) isocyanate(s) monomère(s) de départ, et éventuellement d'autres monomères réactifs avec la fonction isocyanate ;
ii) on fait réagir les monomères de départ, avec un catalyseur de (cyclo)trimérisation dans les conditions de (cyclo)trimérisation ;
iii) on fait réagir le milieu réactionnel de l'étape ii) en présence d'un catalyseur de dimérisation, éventuellement en chauffant le milieu réactionnel à une température d'au moins 40°C ;
iv) on élimine du produit réactionnel de l'étape iii) les monomères de départ n'ayant pas réagi ;
v) éventuellement, on fait réagir le milieu réactionnel avec un agent masquant avant, pendant, ou après les étapes i) à iv) ;
ledit procédé étant **caractérisé en ce que** l'on effectue l'étape iii) jusqu'à un taux de transformation d'au moins 35 %, avantageusement d'au moins 40 % des monomères isocyanates présents dans le milieu réactionnel initial.

20. Procédé selon l'une quelconque des revendications 18 ou 19,
**caractérisé en ce que** le catalyseur de dimérisation est choisi parmi les composés de type tris-(N,N-diatkyl)phosphotriamide, N,N-dialkyleminopyridine ou encore de type trialkylphosphine.

21. Procédé selon l'une quelconque des revendications 18 à 20, **caractérisé en ce que** le catalyseur de dimérisation est choisi parmi les composés de type trialkylphosphine.

22. Procédé selon l'une quelconque des revendications 18 à 21, **caractérisé en ce que** le catalyseur de trimérisation est choisi parmi les composés de type trialkylphosphine.

23. Composition selon l'une quelconque des revendications 1 à 17, pour la réalisation d'un revêtement, **caractérisé par le fait qu'**elle comporte en outre pour addition successive ou simultanée un co-réactif à hydrogène réactif.

24. Utilisation des compositions selon l'une quelconque des revendications 1 à 17, pour la préparation d'un revêtement, notamment une peinture.

25. Procédé de préparation de polymères **caractérisé par le fait qu'**il comporte les étapes suivantes :
- mettre une composition polyisocyanate telle que définie dans l'une quelconque des revendications 1 à 17 en présence d'un co-réactif qui contient des dérivés présentant des hydrogènes réactifs **;**
et
- chauffer le milieu réactionnel ainsi constitué à une température permettant la réticulation des composants.

## Claims

1. Polyisocyanate composition having a mean functionality greater than 3, advantageously greater than 3.5, preferably greater than 4, obtained by polycondensation of diisocyanate or triisocyanate monomers, comprising:
(a) 0.5 to 30% by mass, based on the total mass of components a), b) and c), of compounds carrying a single uretidinedione function having a molecular mass at most equal to twice the mean molecular mass of the isocyanate monomers having the highest molecular mass;
(b) 0.5 to 45% by mass, based on the total mass of components a), b) and c), of compounds carrying a single isocyanurate function and having a molecular mass at most equal to three times the mean molecular mass of said isocyanate monomers having the highest molecular mass;
the molar ratio of (a)/(b) being between 0.02 and 2, advantageously between 0.2 and 1.8, preferably less than or equal to 1.6,
(c) at least 40% by mass, based on the total mass of components a), b) and c), of a mixture of polyisocyanate compounds having a molecular mass at most equal to three times the mean molecular mass of the isocyanate monomers having the lowest molecular mass and carrying at least two isocyanate functions, and
said mixture comprising:
(i) compounds carrying at least two isocyanurate functions, excluding those which comprise uretidinedione functions,
(ii) compounds carrying at least two uretidinedione functions, excluding those which comprise isocyanurate functions, and wherein the number of monomer units is less than 5,
(iii) compounds carrying at least one isocyanurate function and at least one uretidinedione function, having a molecular mass greater than three times the highest molecular mass of the isocyanate monomer compounds above;
said mixture having a ratio of carbonyl functions belonging to a uretidinedione ring /carbonyl functions belonging to an isocyanurate ring + carbonyl functions belonging to a uretidinedione ring of at least 4%;
d) 0 to 25% by mass, based on the mass of components a), b), c), d) and e), of compounds carrying at least one isocyanate function, different from a), b) and c); and
e) 0 to 10% by mass, based on the mass of components a), b), c), d) and e), of impurities.

2. Polyisocyanate composition according to claim 1, **characterised in that** it comprises from 1 to 30% by mass of component (a), based on the total mass of components a) + b) + c).

3. Polyisocyanate composition according to claim 1, **characterised in that** it comprises from 5 to 40% by mass of component (b), based on the total mass of components a) + b) + c).

4. Polyisocyanate composition according to claim 1, **characterised in that** component c) represents at least 45%, advantageously at least 50% by mass, based on the total mass of components a) + b) + c).

5. Polyisocyanate composition according to claim 1, **characterised in that** the mass ratio of [c)(i) + c)(iii)]/b) is greater than 2, advantageously greater than 3, preferably greater than 4.

6. Polyisocyanate composition according to claim 1, **characterised in that** the quantity of compounds c)(ii) is less than 30% by weight, based on all the compounds classified under c), preferably less than 20%, still more preferably less than 15%.

7. Polyisocyanate composition according to claim 1, **characterised in that** the component d) represents at most 10% by mass, based on the total mass of components a) + b) + c) + d) + e).

8. Polyisocyanate composition according to claim 1, **characterised in that** the component e) represents from 0.05% to 10%, generally from 0.1% to 8%, notably at most 5% by mass, based on the total mass of components a) + b) + c) + d) + e).

9. Polyisocyanate composition according to claim 1, **characterised in that** the component e) consists of residues of polycondensation catalyst and/or of by-products of the polycondensation of the starting isocyanate monomers and/or of solvent(s).

10. Polyisocyanate composition according to claim 1, **characterised in that** component d) comprises the said residual isocyanate monomer(s).

11. Polyisocyanate composition according to claim 10, **characterised in that** the said isocyanate monomer(s) represent 0.05% to 20%, more generally from 0.1% to 10%, advantageously at most 2%, preferably at most 1% by mass, based on the mass of components a) + b) + c) + d) + e).

12. Composition according to any one of the preceding claims, **characterised in that** it further comprises a quantity of at most 200%, advantageously at most 100% by mass of a) + b) + c) + d) + e), preferably at most 50% of an organic solvent or mixture of organic solvents which is liquid at ambient temperature, containing no isocyanate function, containing no function capable of reacting with the isocyanate function, having a boiling point of not more than 200°C and being miscible with components a) + b) + c) + d) + e).

13. Composition according to any one of claims 1 to 12, **characterised in that** the compounds comprising at least one uretidinedione ring and at least one isocyanurate ring comprise a group selected from among the following formulae (I) to (V) and mixtures thereof: 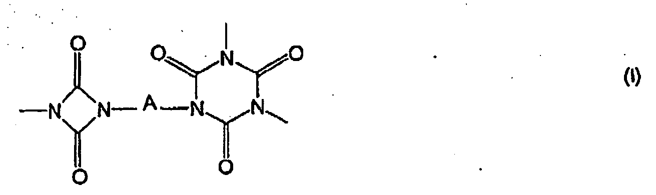 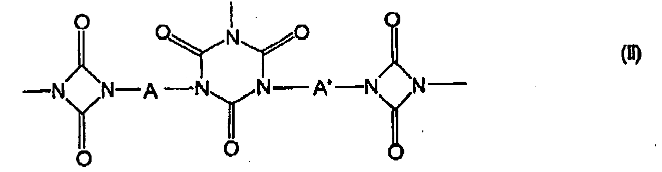 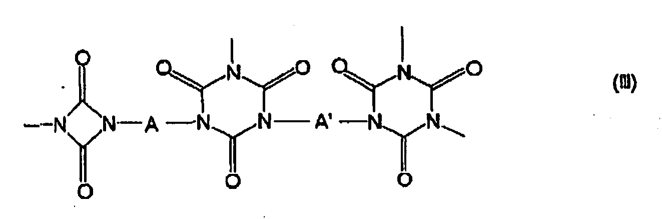 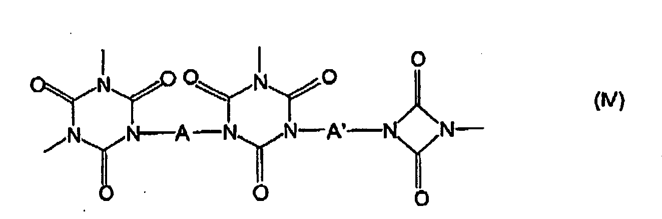 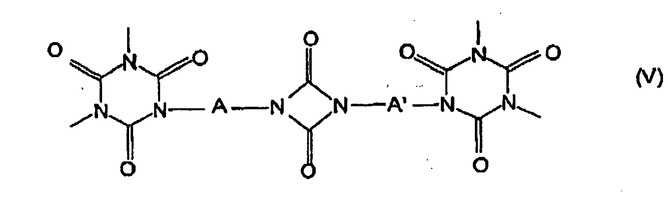 wherein A and A' which may be identical or different represent the residues of a monomeric isocyanate compound after removal of two isocyanate functions.

14. Composition according to any one of the preceding claims, **characterised in that** it contains from 1 to 100%, advantageously from 10 to 100% of the NCO groups present in the composition, masked by a masking agent.

15. Composition according to claim 14, **characterised in that** the masking agent is a monofunctional masking agent selected from among the hydroxylamine derivatives, the oximes, the phenol derivatives, the amide derivatives, the malonates, the ketoesters, the hydroxamates and the nitrogenous heterocyclic compounds.

16. Composition according to claim 15, **characterised in that** the masking agent is methylethylketoxime or methyl pyruvate oxime.

17. Composition according to claim 14, **characterised in that** the masking agent is selected from among the pyrrolyl, 2H-pyrrolyl, imidazolyl, pyrimidinyl, pyridazinyl, pyrazinyl, indolizinyl, isoindolyl, indolyl, indozolyl, purinyl, quinolizinyl, isoquinolyl, pyrazolidinyl, imidazolidinyl and triazolyl groups.

18. Method of preparing a polyisocyanate composition according to one of claims 1 to 17, comprising the following steps:
i) a starting reaction medium is prepared, containing the starting isocyanate monomer(s), and optionally other monomers capable of reacting with the isocyanate function;
ii) the starting reaction medium is reacted in the presence of a dimerisation catalyst, while the reaction medium is optionally heated to a temperature of at least 40°C;
iii) the reaction product from step ii) containing the unreacted monomers is reacted with a (cyclo)trimerisation catalyst, under (cyclo)trimerisation conditions;
iv) the unreacted starting monomers are eliminated from the reaction product of step iii);
v) optionally the reaction medium is reacted with a masking agent before, during or after steps i) to iv);
said process being **characterised in that** step iii) is carried out until a transformation level of at least 35%, advantageously at least 40% of the isocyanate monomers present in the starting reaction medium is achieved.

19. Method of preparing a polyisocyanate composition according to one of claims 1 to 18, comprising the following steps:
i) a starting reaction medium is prepared, containing the starting isocyanate monomer(s), and optionally other monomers capable of reacting with the isocyanate function;
ii) the starting monomers are reacted with a (cyclo)trimerisation catalyst, under (cyclo)trimerisation conditions;
iii) the reaction medium of step ii) is reacted in the presence of a dimerisation catalyst, while the reaction medium is optionally heated to a temperature of at least 40°C;
iv) the unreacted starting monomers are eliminated from the reaction product of step iii);
v) optionally the reaction medium is reacted with a masking agent before, during or after steps i) to iv);
said process being **characterised in that** step iii) is carried out until a transformation level of at least 35%, advantageously at least 40% of the isocyanate monomers present in the starting reaction medium is achieved.

20. Method according to any one of claims 18 or 19, **characterised in that** the dimerisation catalyst is selected from among the compounds of the tris-(N,N-dialkyl)phosphotriamide, N,N-dialkylaminopyridine or trialkylphosphine type.

21. Method according to any one of claims 18 to 20, **characterised in that** the dimerisation catalyst is selected from among the compounds of the trialkylphosphine type.

22. Method according to any one of claims 18 to 21, **characterised in that** the trimerisation catalyst is selected from among the compounds of the trialkylphosphine type.

23. Composition according to any one of claims 1 to 17, for producing a coating, **characterised in that** it further comprises a co-reagent with reactive hydrogen for successive or simultaneous addition.

24. Use of the compositions according to any one of claims 1 to 17, for the preparation of a coating, notably a paint.

25. Method of preparing polymers, **characterised in that** it comprises the following steps:
- bringing a polyisocyanate composition as defined in any one of claims 1 to 17 into the presence of a co-reagent which contains derivatives having reactive hydrogens;
and
- heating the reaction medium thus formed to a temperature at which the components are able to crosslink.

## Patentansprüche

1. Polyisocyanatzusammensetzung mit einer mittleren Funktionalität von über 3, vorteilhafterweise über 3,5, und vorzugsweise über 4, die durch Polykondensation von Diisocyanat- oder Triisocyanatmonomeren erhalten wird und welche:
(a) 0,5 bis 30 Gew.-%, bezogen auf das Gesamtgewicht der Komponenten a), b) und c), Verbindungen, die eine einzige Uretidindionfunktion tragen, und mit einer Molmasse von höchstens gleich dem Zweifachen der mittleren Molmasse der Isocyanatmonomeren mit der höchsten Molmasse,
(b) 0,5 bis 45 Gew.-%, bezogen auf das Gesamtgewicht der Komponenten a), b) und c), Verbindungen, die eine einzige Isocyanuratfunktion tragen, und mit einer Molmasse von höchstens gleich dem Dreifachen der mittleren Molmasse der Isocyanatmonomeren mit der höchsten Molmasse, wobei das Molverhältnis von (a)/(b) 0,02 bis 2, vorteilhafterweise zwischen 0,2 und 1,8, und vorzugsweise weniger als oder gleich 1,6 beträgt,
(c) mindestens 40 Gew.-%, bezogen auf das Gesamtgewicht der Komponenten a), b) und c), eines Gemischs aus Polyisocyanatverbindungen, die eine Molmasse von mindestens gleich dem Dreifachen der mittleren Molmasse der Isocyanatmonomeren mit der kleinsten Molmasse besitzen und mindestens zwei Isocyanatfunktionen tragen, wobei dieses Gemisch:
(i) Verbindungen, die mindestens zwei Isocyanuratfunktionen tragen, ausschließlich derjenigen, die Uretidindionfunktionen enthalten,
(ii) Verbindungen, die mindestens zwei Uretidindionfunktionen tragen, ausschließlich denjenigen, die Isocyanuratfunktionen enthalten, und deren Anzahl der Monomergrundeinheiten weniger als 5 beträgt, und
(iii) Verbindungen, die mindestens eine Isocyanuratfunktion und mindestens eine Uretidindionfunktion tragen und eine Molmasse von höher als dem Dreifachen der höchsten Molmasse der obigen monomeren Isocyanatverbindungen besitzen,
umfasst, und dieses Gemisch einen Anteil der Carbonylfunktionen, die zu einem Uretidindioncyclus gehören/Carbonylfunktionen, die zu einem Isocyanuratcyclus gehören + Carbonylfunktionen, die zu einem Uretidindioncyclus gehören, von mindestens 4 % aufweist,
(d) 0 bis 25 Gew.-%, bezogen auf das Gewicht der Komponenten a), b), c), d) und e), Verbindungen, die mindestens eine Isocyanatfunktion tragen und sich von a), b) und c) unterscheiden, und
(e) 0 bis 10 Gew.-%, bezogen auf das Gewicht der Komponenten a), b), c), d) und e), Verunreinigungen
umfasst.

2. Polyisocyanatzusammensetzung nach Anspruch 1, **dadurch gekennzeichnet, dass** sie 1 bis 30 Gew.-% Komponente (a), bezogen auf das Gesamtgewicht der Komponenten a) + b) + c), enthält.

3. Polyisocyanatzusammensetzung nach Anspruch 1, **dadurch gekennzeichnet, dass** sie 5 bis 40 Gew.-% Komponente (b), bezogen auf das Gesamtgewicht der Komponenten a) + b) + c), enthält.

4. Polyisocyanatzusammensetzung nach Anspruch 1, **dadurch gekennzeichnet, dass** die Komponente c) mindestens 45 Gew.-% und vorteilhafterweise mindestens 50 Gew.-%, bezogen auf das Gesamtgewicht der Komponenten a) + b) + c), ausmacht.

5. Polyisocyanatzusammensetzung nach Anspruch 1, **dadurch gekennzeichnet, dass** das Gewichtsverhältnis [c)(i) + c)(iii)]/b) mehr als 2, vorteilhafterweise mehr als 3, und vorzugsweise mehr als 4 beträgt.

6. Polyisocyanatzusammensetzung nach Anspruch 1, **dadurch gekennzeichnet, dass** die Menge der Verbindungen c) (ii) weniger als 30 Gew.-%, bezogen auf alle unter c) erfassten Verbindungen, vorzugsweise weniger als 20 %, und besonders bevorzugt weniger als 15 % beträgt.

7. Polyisocyanatzusammensetzung nach Anspruch 1, **dadurch gekennzeichnet, dass** die Komponente d) höchstens 10 Gew.-%, bezogen auf das Gesamtgewicht der Komponenten a) + b) + c) + d) + e), ausmacht.

8. Polyisocyanatzusammensetzung nach Anspruch 1, **dadurch gekennzeichnet, dass** die Komponente e) 0,05 bis 10 Gew.-%, im Allgemeinen 0,1 bis 8 Gew.-%, und insbesondere höchstens 5 Gew.-%, bezogen auf das Gesamtgewicht der Komponenten a) + b) + c) + d) + e), ausmacht.

9. Polyisocyanatzusammensetzung nach Anspruch 1, **dadurch gekennzeichnet, dass** die Komponente e) aus Polykondensationsinitiatorrückständen und/oder Polykondensationsnebenprodukten der Ausgangsisocyanatmonomeren und/oder aus Lösungsmittel(n) besteht.

10. Polyisocyanatzusammensetzung nach Anspruch 1, **dadurch gekennzeichnet, dass** die Komponente d) das (die) restliche(n) Isocyanatmonomer(e) umfasst.

11. Polyisocyanatzusammensetzung nach Anspruch 10, **dadurch gekennzeichnet, dass** das (die) Isocyanatmonomer(e) 0,05 bis 20 Gew.-%, ganz allgemein 0,1 bis 10 Gew.-%, vorteilhafterweise höchstens 2 Gew.-%, und vorzugsweise höchstens 1 Gew.-% des Gewichts der Komponenten a) + b) + c) + d) + e) ausmacht (ausmachen).

12. Zusammensetzung nach einem der vorhergehenden Ansprüche, **dadurch gekennzeichnet, dass** sie außerdem eine Menge von höchstens 200 Gew.-% und vorteilhafterweise höchstens 100 Gew.-% von a) + b) + c) + d) + e) und vorzugsweise höchstens 50 % eines organischen Lösungsmittels bzw. eines organischen Lösungsmittelgemischs, das bei Umgebungstemperatur flüssig ist und keine Isocyanatfunktion und keine Funktion, die in der Lage wäre, mit der Isocyanatfunktion zu reagieren, enthält, mit einem Siedepunkt von höchstens 200 °C und welches mit den Komponenten a), b), c), d) und e) mischbar ist, umfasst.

13. Zusammensetzung nach einem der Ansprüche 1 bis 12, **dadurch gekennzeichnet, dass** die Verbindungen, die mindestens einen Uretidindioncyclus und mindestens einen Isocyanuratcyclus enthalten, eine Gruppe umfassen, die aus den folgenden Formeln (I) bis (V) und ihren Gemischen ausgewählt ist: 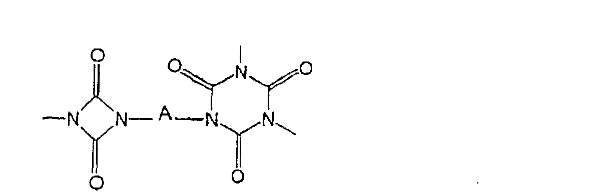 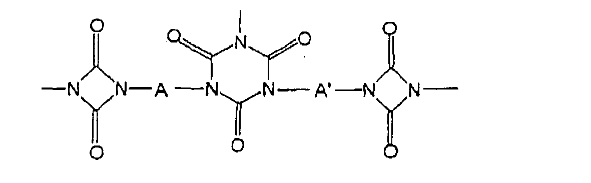 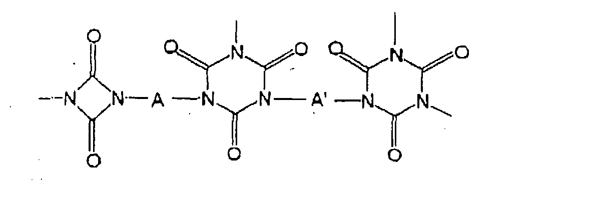 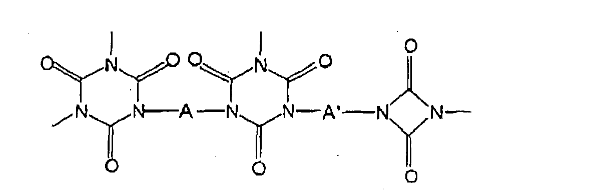 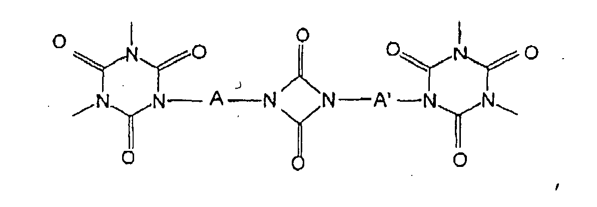 in welchen A und A' gegebenenfalls voneinander verschieden sind und die Reste einer monomeren Isocyanatverbindung nach Entfernung von zwei Isocyanatfunktionen bedeuten.

14. Zusammensetzung nach einem der vorhergehenden Ansprüche, **dadurch gekennzeichnet, dass** sie 1 bis 100 % und vorteilhafterweise 10 bis 100 % NCO-Gruppen, die in der Zusammensetzung von einem Maskierungsmittel maskiert vorliegen, umfasst.

15. Zusammensetzung nach Anspruch 14, **dadurch gekennzeichnet, dass** das Maskierungsmittel ein monofunktionelles Maskierungsmittel ist, das aus Hydroxylaminderivaten, Oximen, Phenolderivaten, Amidderivaten, Malonaten, Ketoestern, Hydroxamaten und Stickstoff enthaltenden heterocyclischen Verbindungen ausgewählt ist.

16. Zusammensetzung nach Anspruch 15, **dadurch gekennzeichnet, dass** das Maskierungsmittel Methylethylketoxim oder Methylpyruvatoxim ist.

17. Zusammensetzung nach Anspruch 14, **dadurch gekennzeichnet, dass** das Maskierungsmittel aus der Pyrolyl-, 2H-Pyrrolyl-, Imidazolyl-, Pyrimidinyl-, Pyridazinyl-, Pyrazinyl-, Indolizinyl-, Isoindolyl-, Indolyl-, Indozolyl-, Purinyl-, Chinolizinyl-, Isochinolyl-, Pyrazolidinyl-, Imidazolidinyl- und Triazolylgruppe ausgewählt ist.

18. Verfahren zur Herstellung einer Polyisocyanatzusammensetzung nach einem der Ansprüche 1 bis 17, das die Stufen:
i) Herstellen eines Ausgangsreaktionsmediums, das das (die) Ausgangsisocyanatmönomer(e) und gegebenenfalls weitere mit der Isocyanatfunktion reaktionsfähige Monomere umfasst,
ii) Umsetzen des Ausgangsreaktionsmediums in Gegenwart eines Dimerisationsinitiators, gegebenenfalls indem das Reaktionsmedium auf eine Temperatur von mindestens 40 °C erhitzt wird,
iii) Umsetzen des Reaktionsproduktes von Stufe ii), das Monomere enthält, die nicht mit einem (Cyclo-)Trimerisationsinitiator reagiert haben, unter (Cyclo-)Trimerisationsbedingungen,
iv) Entfernen der nicht umgesetzten Ausgangsmonomeren aus dem Reaktionsprodukt von Stufe iii) und gegebenenfalls
v) Umsetzen des Reaktionsmediums mit einem Maskierungsmittel vor, während oder nach den Stufen i) bis iv)
umfasst, wobei das Verfahren **dadurch gekennzeichnet ist, dass** die Stufe iii) bis zu einem Umsatz von mindestens 35 % und vorteilhafterweise von mindestens 40 % der in dem anfänglichen Reaktionsmedium vorhandenen Isocyanatmonomeren durchgeführt wird.

19. Verfahren zur Herstellung einer Polyisocyanatzusammensetzung nach einem der Ansprüche 1 bis 18, das die Stufen:
i) Herstellen eines Ausgangsreaktionsmediums, das das (die) Ausgangsisocyanatmonomer(e) und gegebenenfalls weitere mit der Isocyanatfunktion reaktionsfähige Monomere umfasst,
ii) Umsetzen der Ausgangsmonomeren mit einem (Cyclo-)Trimerisationsinitiator unter (Cyclo-)Trimerisationsbedingungen,
iii) Umsetzen des Reaktionsmediums von Stufe ii) in Gegenwart eines Dimerisationsinitiators, gegebenenfalls indem das Reaktionsmedium auf eine Temperatur von mindestens 40 °C erhitzt wird,
iv) Entfernen der nicht umgesetzten Ausgangsmonomeren aus dem Reaktionsprodukt von Stufe iii) und gegebenenfalls
v) Umsetzen des Reaktionsmediums mit einem Maskierungsmittel vor, während oder nach den Stufen i) bis iv)
umfasst, wobei das Verfahren **dadurch gekennzeichnet ist, dass** die Stufe iii) bis zu einem Umsatz von mindestens 35 % und vorteilhafterweise von mindestens 40 % der in dem anfänglichen Reaktionsmedium vorhandenen Isocyanatmonomeren durchgeführt wird.

20. Verfahren nach Anspruch 18 oder 19, **dadurch gekennzeichnet, dass** der Dimerisationsinitiator aus Verbindungen vom Typ Tris(N,N-dialkyl)phosphotriamid, N,N-Dialkylaminopyridin oder auch Trialkylphosphin ausgewählt wird.

21. Verfahren nach einem der Ansprüche 18 bis 20, **dadurch gekennzeichnet, dass** der Dimerisationsinitiator aus Verbindungen vom Typ Trialkylphosphin ausgewählt wird.

22. Verfahren nach einem der Ansprüche 18 bis 21, **dadurch gekennzeichnet, dass** der Trimerisationsinitiator aus Verbindungen vom Typ Trialkylphosphin ausgewählt wird.

23. Zusammensetzung nach einem der Ansprüche 1 bis 17 für die Herstellung einer Beschichtung, **dadurch gekennzeichnet, dass** sie außerdem für eine aufeinanderfolgende oder gleichzeitige Zugabe einen Co-Reaktanten mit reaktionsfähigem Wasserstoff umfasst.

24. Verwendung der Zusammensetzungen nach einem der Ansprüche 1 bis 17 zur Herstellung einer Beschichtung und insbesondere eines Anstrichs.

25. Verfahren zur Herstellung von Polymeren, **dadurch gekennzeichnet, dass** es die Stufen:
- Zusammenbringen einer in einem der Ansprüche 1 bis 17 definierten Polyisocyanatzusammensetzung mit einem Co-Reaktanten, der reaktionsfähige Wasserstoffatome aufweisende Derivate umfasst, und
- Erhitzen des so gebildeten Reaktionsmediums auf eine Temperatur, welche die Vernetzung der Komponenten erlaubt,
umfasst.
